# EUROPEAN PATENT SPECIFICATION

(11) **EP 0 406 297 B1**
(45) Date of publication and mention of the grant of the patent: **28.08.1996**
(21) Application number: 89904170.1
(22) Date of filing: 21.03.1989
(51) Int. Cl.: A61K 38/08

(54) **Medical use of neuropeptide Antagonist G**
Medizinische Verwendung von neuropeptid Antagonist G
L'utilisation médicale d'Antagoniste G des neuropeptides

(30) Priority: 21.03.1988 GB 8806644
(43) Date of publication of application: 09.01.1991
(73) Proprietor: IMPERIAL CANCER RESEARCH TECHNOLOGY LIMITED, Surrey CR9 2PL (GB)
(72) Inventor: ROZENGURT, Enrique, Lincoln's Inn Fields London WC2 (GB); WOLL, Penella, Lincoln's Inn Fields London WC2 (GB)
(74) Representative: Goldin, Douglas Michael
(86) International application number: GB8900300
(87) International publication number: WO8909232

(56) References cited:
- WO-A-88/07551
- The Journal of Biological Chemistry, volume 262, no. 9, 25 March 1987, The American Society of Biological Chemists, Inc. (US), I. Zachary et al.: "Identification of a receptor for peptides of the bombesin family in Swiss 3T3 cells by affinity cross-linking", pages 3947-3950 see the whole article cited in the application
- Proc. Natl. Acad. Sci. USA, volume 85, March 1988, P.J. Woll et al.: (D-Arg1, D-Phe5, D-Trp7,9,Leu11) substance P, a potent bombesin antagonist in murine Swiss 3T3 cells, inhibits the growth of human small cell lung cancer cells in vitro", pages 1859-1863 see the whole article, especially figure 1 - cited in the application
- The Journal of Biological Chemistry, volume 262, no. 23, 15 August 1987, The American Society for Biochemistry and Molecular Biology, Inc., (US), R.M. Kris et al.: "Identification of the bombesin receptor on murine and human cells by cross-linking experiments", pages 11215-11220 see the whole article cited in the application
- Chemical Abstracts, volume 109, no. 1, 4 July 1988, (Columbus, Ohio, US), K.D. Brown et al.: "Characterization of the high-affinity receptors on Swiss 3T3 cells which mediate the binding, internalization and degradation of the mitogenic peptide bombesin", see pages 111-112, abstract 1169t, & Biochem. J. 1988, 252(1), 227-35
- The Journal of Biological Chemistry, volume 263, no. 6, 25 February 1988, The American Society for Biochemistry and Molecular Biology, Inc., (US), J.B. Fischer et al.: "The bombesin receptor is coupled to a guanine nucleotide-binding protein which is insensitive to pertussis and cholera toxins", pages 2808-2816 see page 2809, column 2, line 2 - page 2810, column 1, line 4
- Biological Abstracts/RRM, no. 35054560, A.Sundan: "Identification of the bombesin receptor by monoclonal anti-idiotypic antibodies", see the title and terms, & Symposium on Growth Factors and their Receptors: Genetic Control and Rational Application held at the 17th Annual Meeting of the UCLA (University of California-Los Angelos) Symposia on Molecular and Cellular Biology, Keystone, Colorado, USA, January 24-30,1988, J Cell Biochem Suppl. 1988. vol 0, no 12 part A p116
- Chemical Abstracts, volume 110, no. 11, 13 March 1989, (Columbus, Ohio, US), J. Sinnett-Smith et al.: "Characterization of a bombesin receptor on Swiss mouse 3T3 cells affinity cross-linking", see page 124, abstract 89048g, & J.Cell. Biochem. 1988, 38(4), 237-49

## Description

THIS INVENTION relates to an antagonists to various receptors for neuropeptides, in particular to antagonist G and its use in the treatment of small cell lung cancer.

Neuropeptides are increasingly implicated in the control of cell proliferation¹. The amphibian tetradecapeptide bombesin and its mammalian homologue, gastrin-releasing peptide (GRP) are potent mitogens for mouse fibroblasts² and may act as autocrine growth factors for small cell lung cancer³.

In our International Patent Application WO88/07551 we describe certain new peptides that are receptors for certain peptides of the bombesin family. We also describe antagonists to the bombesin receptors including two known compounds that we have found to be particularly valuable as bombesin receptor antagonists. These antagonists are analogues of an 11-mer neuropeptide called substance P which is of interest in studies in pain transmission. Substance P has the formula:
Arg-Pro-Lys-Pro-Gln-Gln-Phe-Phe-Gly-Leu-Met-NH₂

A commercially available analogue of Substance P, that we call antagonist A, has the formula:
D-Arg-D-Pro-Lys-Pro-Gln-Gln-D-Trp-Phe-D-Trp-Leu-Leu-NH₂

A further commercially available structural variant of substance P which we have disclosed in our above-mentioned Patent Application is antagonist D which has the formula:
D-Arg-Pro-Lys-Pro-D-Phe-Gln-D-Trp-Phe-D-Trp-Leu-Leu-NH₂

In our above-mentioned Patent Application, we have described how our work with antagonist A and antagonist D has drawn to our attention the importance of position 5 in the amino acid sequence of antagonist A and antagonist D and we have referred to valuable further antagonists which are amino acid position 5 variants of antagonist A and antagonist D where position 5 contains a D-Trp, D-Tyr or Me-Phe group.

In the work described in our above-mentioned Patent Application, we suggested that the various antagonists that we have described acted as antagonists by binding the ligand binding site on the receptor and that by so doing, the compounds were of medical interest in that they would be able to influence cell proliferation that occurred under the influence of the neuropeptide/receptor interactions.

Our further research work has led us to the conclusion that while the various antagonists that we have described in our above-mentioned Patent Application do bind to the receptor, and hence are of medical interest for the reason mentioned, the binding is not at the ligand binding site but is at a different binding site on the receptor. We reached this conclusion from our further researches that indicate that our antagonists will bind not only with the bombesin receptors of the type described in our above-mentioned Patent Application but also with other neuropeptide receptors that, in common with the bombesin receptor, use the inositol signalling pathway. There are at least two further such receptors, the bradykinin receptor and the vasopressin receptor and antagonists A and D have been found capable of binding with these receptors. This particular property of antagonists A and D is in complete contrast to the properties of bombesin itself which, by definition will bind to the bombesin receptors but will not bind to the vasopressin or bradykinin receptors. This shows that the binding site of antagonist A and D on the bombesin receptor is not the ligand binding site but is in fact probably a separate conserved domain which is also to be found in the other receptors using the inositol signalling pathway.

Our most recent discovery therefore increases the medical importance of not only antagonists A and D but also analogues thereof such as antagonist G in that they are able to influence not only cell proliferation occurring under the influence of bombesin-like peptides but cell proliferation occurring under the influence of a much wider range of mitogenic neuropeptides.

Accordingly, the present invention provides antagonist G for use in a method of treatment of the human or animal body, in particular in a method of influencing cell proliferation that is influenced by neuropeptides that normally bind to receptors that use the inositol signalling pathway. Antagonist G is particularly of use in a method of treatment of small cell lung cancer.

Antagonist G is of formula:
Arg.D-Trp.MePhe.D-Trp-Leu-Met-NH₂.

Additionally Antagonist G is of interest in the retarding or abolition of the growth of small cell lung cancer and, when labelled, in the diagnosis, in vitro or in vivo of small cell lung cancer. Such labelling may be direct on the antagonist molecule, or indirect through a molecule that binds to the antagonist.

Included within the scope of this invention is antagonist G for use in the production of a medicament for influencing cell proliferation influenced by neuropeptides that normally bind to receptors that use the inositol signalling pathway and particularly antagonist G for use in the production of a medicament for the treatment of or diagnosis of small cell lung cancer. For this purpose antagonist G can be administered parenterally in an effective dose to a host in need of such treatment or diagnosis.

### Description of the Drawings

Figures 1 and 2 illustrate results of experiments described in the Comparative Example.

Figures 3 to 5 illustrate the results of the inhibitory effect of antagonists A, D and G on small cell lung cancer cell lines as described in Example 1.

The following Examples are given to illustrate the invention.

### COMPARATIVE EXAMPLE

Swiss 3T3 cells are widely available for experimental use and are available from the American Type Culture Collection in Rockville, Maryland, U.S.A., under the Deposit No. ATCC-CCL92.

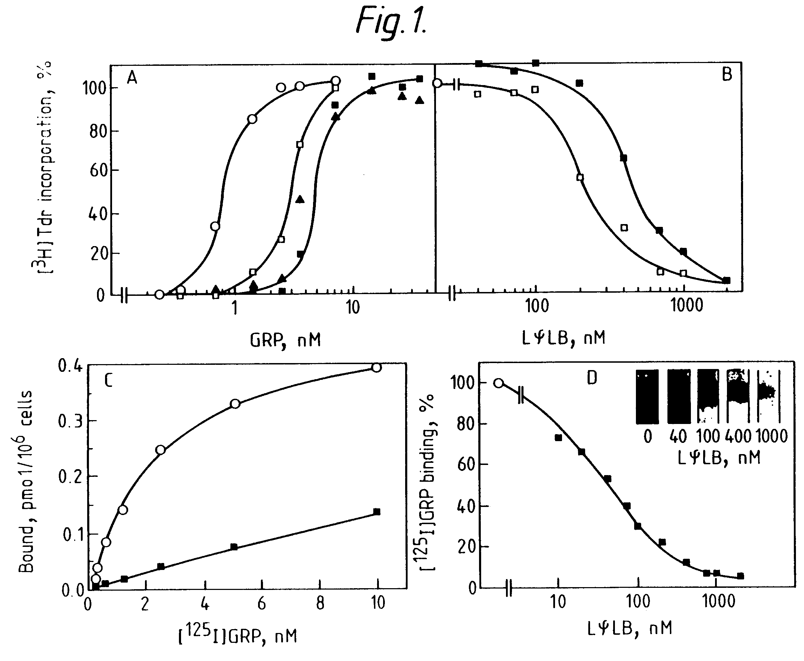

The pseudopeptide [Leu¹³-ψ(CH₂NH)Leu¹⁴]bombesin was synthesised during a systematic study of peptide backbone modifications in bombesin analogues⁵. It was shown to cause 50% inhibition (IC₅₀) of bombesin-stimulated amylase release from guinea pig pancreatic acini at 35 nM. We have now characterised its mode of action in Swiss 3T3 cells. DNA synthesis, measured by [³H]thymidine incorporation into quiescent cells⁶ in the presence of insulin 1 µg/ml and varying concentrations of GRP was strongly inhibited by [Leu¹³-ψ(CH₂NH)Leu¹⁴-bombesin with an effect at 1 µM equivalent to that of 20 µM [DArg¹,DPhe⁵,DTrp^{7,9},Leu¹¹]substance P i.e. Substance D (Figure 1A). The IC₅₀ for [Leu¹³-ψ(CH₂NH)Leu¹⁴]bombesin was 455 nM in the presence of 3.6 nM GRP (Fig. 1B). In addition [Leu¹³-ψ(CH₂NH)Leu¹⁴]bombesin blocked the earliest cellular events elicited by GRP⁷, including Ca²⁺ mobilisation (5 secs) and epidermal growth factor (EGF) receptor transmodulation (1 min), which is dependent on protein kinase C activation. [Leu¹³-ψ(CH₂NH)Leu¹⁴]bombesin inhibited the specific binding of [¹²⁵I]GRP to the cells (Fig. 1C and D) and blocked cross-linking of [¹²⁵I]GRP to the Mr 75,000-85,000 glycoprotein component of the receptor (Fig. 1D, inset)^{8,9}. Thus, [Leu¹³-ψ(CH₂NH)Leu¹⁴]bombesin is a potent bombesin antagonist in Swiss 3T3 cells, acting at receptor level.

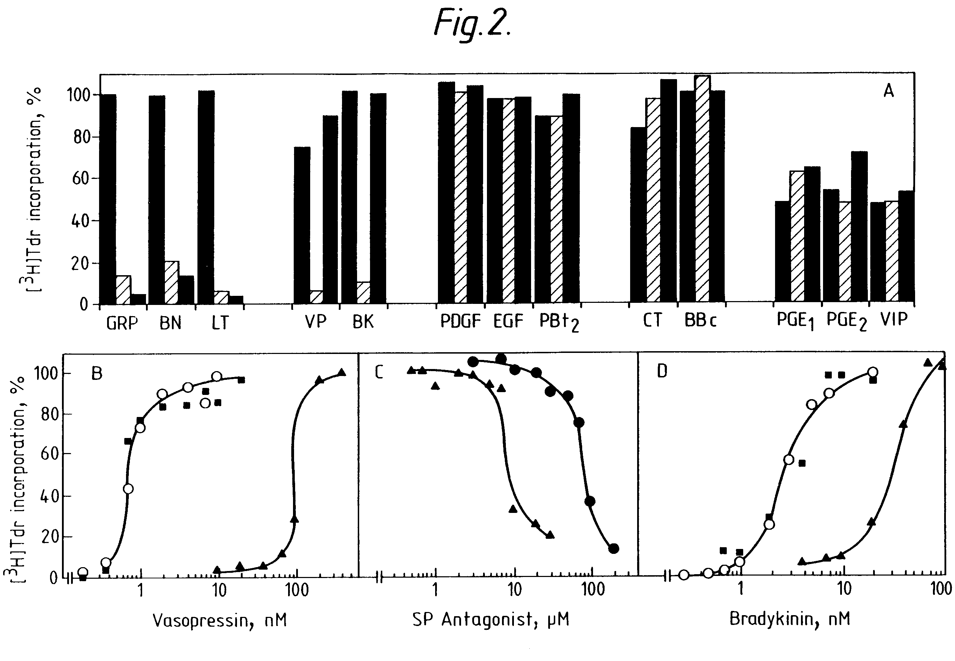

The tachykinin substance P has minimal amino acid sequence homology with bombesin and neither inhibits the binding of [¹²⁵I]GRP nor stimulates DNA synthesis in Swiss 3T3 cells^{2,10}. Our earlier-mentioned Patent Application and references 10, 11 describe how antagonist A is a bombesin antagonist and that antagonist D is 5-10 fold more potent than antagonist A. Both also inhibit vasopressin-stimulated DNA synthesis and [³H]vasopressin binding in Swiss 3T3 cells ^{4,12,13}. Because of this, we have tested the new bombesin antagonists rigorously to establish their specificity against mitogens acting through different signal transduction pathways (Fig. 2A). As expected, antagonist D and [Leu¹³-ψ(CH₂NH)Leu¹⁴]bombesin were both effective against various bombesin-like peptides. Neither of them inhibited mitogenesis stimulated by the polypeptide growth factors EGF and platelet-derived growth factor (PDGF), the protein kinase C activator phorbol dibutyrate, or the cAMP elevators cholera toxin and 8-bromo-cAMP. Although antagonist D was a potent inhibitor of vasopressin-induced DNA synthesis, [Leu¹³-ψ(CH₂NH)Leu¹⁴]bombesin had no effect at concentrations that strongly inhibited GRP-induced mitogenesis (Fig. 2B). This dramatic difference in specificity prompted us to compare the effects of antagonist D and [Leu¹³-ψ(CH₂NH)Leu¹⁴]bombesin on other mitogenic neuropeptides.

Bradykinin, like bombesin and vasopressin, stimulates calcium mobilisation and inositol phosphate turnover in several cell types¹⁴⁻¹⁷. We found that in Swiss 3T3 cells loaded with the fluorescent Ca²⁺ indicator Fura-2, the addition of bradykinin caused a rapid and transient increase in the cytosolic Ca²⁺ concentration with kinetics similar to those obtained with bombesin and vasopressin but distinct from those of PDGF¹⁸. Bradykinin did not inhibit the binding of either [³H]vasopressin or [¹²⁵I]GRP to Swiss 3T3 cells, indicating that their receptors are distinct. In the presence of insulin, bradykinin caused maximal stimulation of DNA synthesis in these cells (Figs. 2C & D) in contrast to its weak mitogenic effect in human fibroblasts^{19,20}. Thus, bradykinin provided a novel mitogen with which to test the new antagonists. Mitogenesis induced by 9.4 nM bradykinin was inhibited by antagonist A and antagonist D (Figs. 2C & D) with IC₅₀ of 90 µM and 8.3 µM respectively which is the same relative potency obtained with GRP⁴ and vasopressin (data not shown). Remarkably [Leu¹³-ψ(CH₂NH)Leu¹⁴]bombesin had no effect on bradykinin-stimulated DNA synthesis (Fig. 2C). While bombesin, bradykinin and vasopressin activate the inositol phosphate signalling pathway, vasoactive intestinal peptide (VIP) is a neuropeptide that stimulates DNA synthesis via cAMP²¹. Neither antagonist D nor [Leu¹³-ψ(CH₂NH)Leu¹⁴]bombesin inhibited mitogenesis stimulated by VIP (Fig. 2A).

The striking difference in specificity between [Leu¹³-ψ(CH₂NH)Leu¹⁴]bombesin and the substance P antagonists presented here has important implications. The substance P antagonists block the mitogenic responses elicited through three distinct receptors, bombesin/GRP, bradykinin and vasopressin. They do not block those elicited by other mitogens which act through different transmembrane signalling pathways including cAMP and protein kinase C activation. The substitution of the two amino acids distinguishing antagonist D from antagonist A causes a consistent 5-10 fold increase in potency against each mitogen, which suggests that these antagonists recognise a common site in their distinct receptors. As bombesin, bradykinin, vasopressin and the substance P antagonists are structurally unrelated this putative binding site cannot be the ligand recognition site. Thus, we believe that antagonist D and antagonist A recognize a conserved domain on each of the receptors. Since a common function of these receptors is the induction of inositol polyphosphate formation and Ca²⁺ mobilisation, we believe that the substance P antagonists could bind with a region on each receptor that is essential for coupling with G proteins involved in Ca²⁺ signalling²². Our theories require that an antagonist structurally related to one of these mitogens should bind to its discrete ligand-recognition site causing specific inhibition of its biological effects but not those of the other mitogenic neuropeptides. Our results demonstrate that the pseudopeptide antagonist [Leu¹³- (CH₂NH)Leu¹⁴]bombesin satisfies these criteria.

### EXAMPLE 1

The ability of antagonists A, D and G to inhibit the growth in vitro of a small cell lung cancer (SCLC) cell line was tested. The cell lines used were
a) H69 and H128 from the ATCC in Maryland USA;
b) H209, H345 and H510A obtained from Dr. A. Gazdar at NCI, NIH, Maryland USA;
c) UCH25 from Dr. P. Beverley, University College, London, England.

Antagonist A when tested was found effective in inhibiting growth of SCLC at concentrations of 50-150 µM in 5 of 5 cell lines tested: H69, H82, H128, H417 and UCH25.

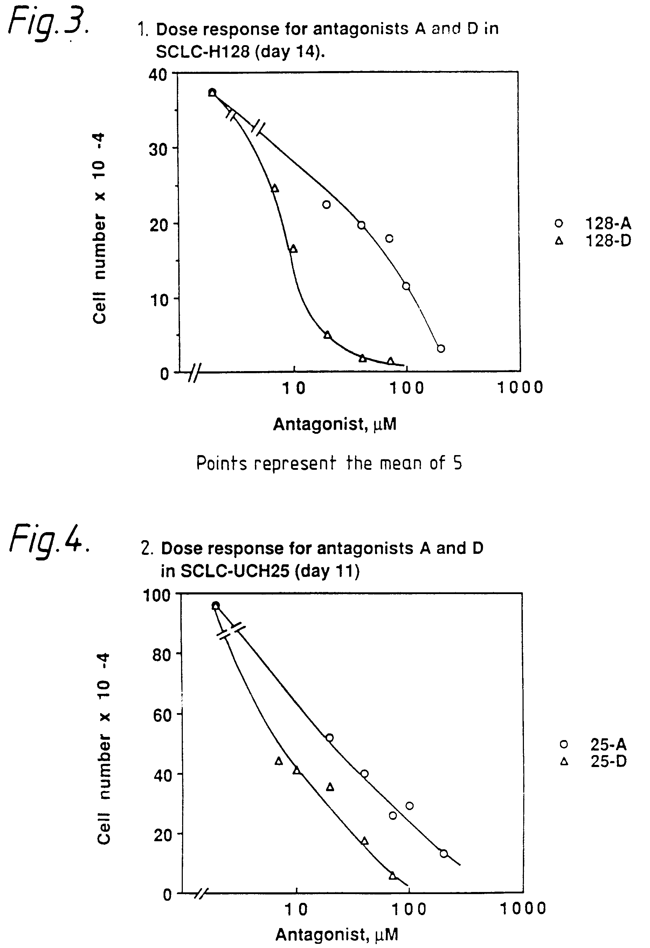

Antagonist D was found to be consistently 5-10 fold more potent than Antagonist A in inhibiting growth of SCLC, see Figures 3 and 4. Antagonist D abolishes cell growth at 40-50 µM and is active in 6 of 6 cell lines tested: H69, H128, H209, H345, H510A and UCH25.

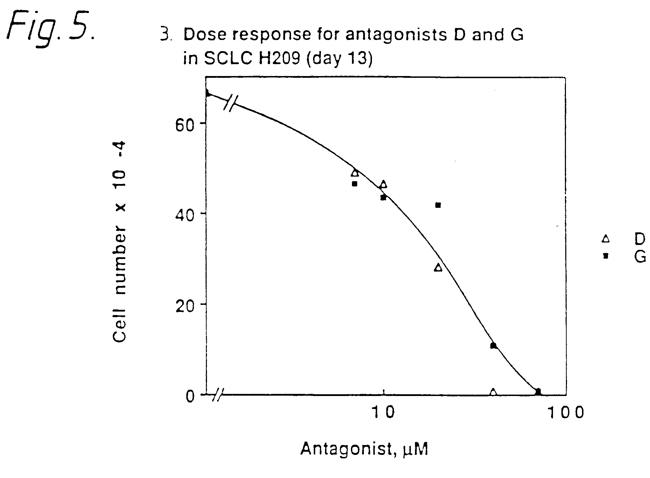

Antagonist G was found to be as potent as Antagonist D as an antagonist of vasopressin induced cell growth in Swiss 3T3 cells, but less potent as an antagonist of growth induced by either bradykinin or GRP. In SCLC cells antagonist G is equipotent with antagonist D in terms of inhibition of growth, see Figure 5. This activity has been demonstrated in 4 of 4 cell lines tested: H69, H209, H345 and H510A.

In calcium mobilisation tests, transient elevation of intracellular [Ca 2+] has been demonstrated in several SCLC cell lines in response to bradykinin, GRP and vasopressin. These responses can be blocked by 20 µM antagonist D in 3 of 3 lines tested (H69, H345, H510A) and by 20 µM antagonist G in H510A.

### References

1. Zachary, I., Woll, P.J. & Rozengurt, E. Devel. Biol. 124, 295-308 (1987).
2. Rozengurt, E. & Sinnett-Smith, J. Proc. natn. Acad. Sci. USA. 80, 2936-2940 (1983).
3. Cuttitta, F. et al. Nature 316, 823-826. (1985)
4. Woll, P.J. & Rozengurt, E. Proc. natn. Acad. Sci. USA. in press.
5. Coy, D.H. et al. J. Biol. Chem. 263, 5056-5060 (1988)
6. Dicker, P. & Rozengurt, E. Nature 287, 607-612 (1980).
7. Rozengurt, E. Science 234, 161-166 (1986).
8. Zachary, I. & Rozengurt, E. J. Biol. Chem. 262, 3947-3950 (1987).
9. Sinnett-Smith, J., Zachary, I. & Rozengurt, E. J. Cell. Biochem. 38, 237-249 (1988)
10. Zachary, I. & Rozengurt, E. Proc. natn. Acad. Sci. USA. 82, 7616-7620 (1985).
11. Jensen, R.T., Jones, S.W., Folkers, K. & Gardner, J.D. Nature 309, 61-63 (1984).
12. Corps, A.N., Rees, L.H. & Brown, K.D. Biochem. J. 231, 781-784 (1985).
13. Zachary, I. & Rozengurt, E. Biochem. Biophys. Res. Commun. 137, 135-141 (1986).
14. Burch, R.M. & Axelrod, J. Proc. natn. Acad. Sci. USA. 84, 6374-6378 (1987).
15. Jackson, T.R., Hallam, T.J., Downes, C.P. & Hanley, M.R. EMBO J. 6, 49-54 (1987).
16. Osugi, T., Imaizumi, T., Mizushima, A., Uchida, S. & Yoshida, H. J. Pharmacol. Exp. Ther. 240, 617-622 (1987).
17. Tilly, B.C. et al. Biochem. J. 244, 129-133 (1987).
18. Lopez-Rivas, A., Mendoza, S.A., Nånberg. E., Sinnett-Smith, J., & Rozengurt, E. Proc. natn. Acad. Sci. USA. 84, 5768-5772 (1987).
19. Owen, N.E. & Villereal, M.L. Cell 32, 979-985 (1983).
20. Coughlin, S.R., Lee, W.M.F., Williams, P.W., Giels, G.M. and Williams, L.T. Cell 43, 243-251 (1985).
21. Zurier, R.B., Kozma, M., Sinnett-Smith, J. & Rozengurt, E. Exp. Cell Res. in press.
22. Stryer, L. & Bourne, H.R. Ann. Rev. Cell Biol. 2, 391-419 (1986).

### Figure Legends

Figure 1: Effects of [Leu¹³-ψ(CH₂NH)Leu¹⁴]bombesin (LψLB) in murine Swiss 3T3 cells. A. Dose-response curves for GRP-induced DNA synthesis with 1 µCi/ml (1 µm) [³H]thymidine ([³H]Tdr) and 1 µg/ml insulin alone (o) or with 500 mM (□) or 1 µM (■) LψLB, or 20 µM Antagonist D (△). Values are expressed as a percentage of [³H]Tdr incorporation obtained with 10% fetal calf serum. B. Dose-response curves for LψLB inhibition of DNA synthesis induced by 2.4 nM (□) and 3.6 nM (■) GRP with 1 µg/ml insulin and 1 µCi/ml [³H]Tdr. C. Concentration-dependence of [¹²⁵I]GPR binding to Swiss 3T3 cells at 37^{o}C. Specific cell-associated binding is shown in the absence (o) or presence (■) or 500 nM LψLB. D. Inhibition of specific [¹²⁵I]GRP binding to Swiss 3T3 cells by LψLB (■) expressed as a percentage of binding obtained with 1 nM [¹²⁵I]GRP in the absence of antagonist (o). Inset. Effect of LwLB on the affinity-labelling of the Mr 75-85000 bombesin receptor-associated protein.

Methods: Swiss 3T3 cells were maintained in culture and assays of DNA synthesis performed as previously described ^{2,4,6}. In all the figures, values shown represent the mean of at least 2 determinations. For [¹²⁵I]GRP binding studies, confluent and quiescent cells were washed twice with Dulbecco's modified Eagle's medium then incubated at 37^{o}C in 0.75 ml of binding medium¹⁰ containing the stated concentration of [¹²⁵I]GRP and LψLB. Cell-associated [¹²⁵I]GRP binding was measured after 30 mins. Non-specific binding was determined by the addition of 500-fold excess of unlabelled GRP. For crosslinking studies, confluent and quiescent cells were washed twice with binding medium⁸ then incubated at 24^{o}C in 1 ml of binding medium (pH 7.0) containing 1 nM [¹²⁵I]GRP and various concentrations of LψLB. After 10 mins they were washed twice with binding medium, then incubated at 24^{o}C in 1 ml containing 6mM ethylene glycol bis(succinimidylsuccinate) at pH 7.4. After 10 mins they were washed twice with cold binding medium and solubilised in 100 µl sample buffer⁸, then immediately boiled for 5 mins, and electrophoresed on a 10% polyacrylamide gel.

Figure 2. Specificity of the bombesin antagonists.
A. DNA synthesis with a variety of mitogens measured (as in Fig. 1) in the presence of 1 µCi/ml [³HTdr] and 1 µg/ml insulin alone (■) or with 20 µM antagonist D or 1 µm LψLB (□). Mitogens were used at the following concentrations: GRP, 3.6 nM; bombesin (BN), 1.2 nM; litorin (LT), 1.8 nM, vasopressin (VP), 9.2 nM; bradykinin (BK), 9.4 nM; PDGF, 1 nM; EGF, 0.4nM; Phorbol 12,13-dibutyrate (PBt₂), 50 ng/ml; cholera toxin (CT), 100 ng/ml with isobutylmethylxanthine (IBMX), 10 µM; 8-bromo-cAMP (8Bc), 2.5 mM; prostaglandin E₁ (PGE₁), 100 ng/ml with IBMX, 10 µM; prostaglandin E₂ (PGE₂), 200 ng/ml with IBMX, 10 µM; VIP, 3.0 nM with4-(3-butoxy-4-methoxybenzyl)-2-imidozolidine, 5 µM. Values are expressed as a percentage of [³H]Tdr incorporation obtained with 10% fetal calf serum.
B. Dose-response curves for vasopressin-induced DNA synthesis with 1 µCi/ml [³H]Tdr and 1 µg/ml insulin alone (o) or with 1 uM LψLB (■) or 20 µM antagonist D (□).
C. Inhibition of DNA synthesis stimulated by 9.4 nM bradykinin with 1 µCi/ml [³H]Tdr and 1 µg/ml insulin, by various concentrations of antagonist D (△) or Antagonist A (o). D. Dose-response curves for bradykinin-induced DNA synthesis with 1 µC/ml [³H]Tdr and 1 µg/ml insulin alone(o) or with 1 µM LψLB (■) or 20 µM antagonist D (△).

### Footnote 1: List of Abbreviations

- PBS: - phosphate buffered saline
- BSA: - bovine serum albumin
- GRP: - gastrin releasing peptide
- EGS: - ethyleneglycolbis(succimidylsuccinate)
- DSS: - disuccinimidyl suberate
- EGTA: - ethylene bis (oxyethylenenitrilo) tetraacetic acid
- Hepes: - 4-(2-hydroxyethyl)-1-piperazineethanesulfonic acid
- SDS-PAGE: - sodium dodecyl sulfate-polyacrylamide gel electrophoresis
- VIP: - vasoactive intestinal peptide
- PDGF: - platelet-derived growth factor
- FGF: - fibroblast growth factor
- EGF: - epidermal growth factor
- PBt₂: - phorbol 12,13-dibutyrate

## Claims

1. Antagonist G (Arg-DTrp-MePhe-DTrp-Leu-Met-NH₂) for use in a method of treatment of the human or animal body.

2. Use of antagonist G (Arg-DTrp-MePhe-DTrp-Leu-Met-NH₂) for the manufacture of a medicament for the treatment of or diagnosis of small cell lung cancer.

## Patentansprüche

1. Antagonist G (Arg-DTrp-MePhe-DTrp-Leu-Met-NH₂) zur Verwendung in einem Verfahren zur Behandlung des menschlichen oder tierischen Körpers.

2. Verwendung von Antagonist G (Arg-DTrp-MePhe-DTrp-Leu-Met-NH₂) für die Herstellung eines Medikaments zur Behandlung oder zur Diagnose von kleinzelligem Lungenkrebs.

## Revendications

1. Antagoniste G (Arg-DTrp-MePhe-DTrp-Leu-Met-NH₂) pour utilisation dans une méthode de traitement du corps humain ou animal.

2. Utilisation de l'antagoniste G (Arg-DTrp-MePhe-DTrp-Leu-Met-NH₂) pour la préparation d'un médicament pour le traitement du cancer du poumon à petites cellules ou le diagnostic dudit cancer.
